# EUROPÄISCHE PATENTSCHRIFT

(11) **EP 4 059 939 B1**
(45) Veröffentlichungstag und Bekanntmachung des Hinweises auf die Patenterteilung: **07.06.2023**
(21) Anmeldenummer: 21163474.6
(22) Anmeldetag: 18.03.2021
(51) Int. Cl.: C07F 9/6574, C07C 45/50

(54) **BISPHOSPHITLIGANDEN AUF BASIS VON BENZPINAKOL**
BISPHOSPHITE LIGANDS BASED ON BENZOPINACOL
LIGANDS BISPHOSPHITE À BASE DE BENZOPINACOL

(43) Veröffentlichungstag der Anmeldung: 21.09.2022
(73) Patentinhaber: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); MARKOVIC, Ana, 45721 Haltern am See (DE); KUCMIERCZYK, Peter, 44628 Herne (DE); KNOSSALLA, Johannes, 46514 Gahlen (DE); SELENT, Detlef, 18059 Rostock (DE); BÖRNER, Armin, 18059 Rostock (DE); ROMEIKE, Kerstin, 18109 Rostock (DE)
(74) Vertreter: Evonik Patent Association

(56) Entgegenhaltungen:
- EP-A1- 3 029 044
- WO-A2-2005/042458

## Beschreibung

Die vorliegende Erfindung betrifft Bisphosphitliganden auf Basis von Benzpinakol, sowie deren Verwendung in der Hydroformylierung.

In EP 3029044 A1 werden Bisphosphite beschrieben, welche einen unsymmetrischen Biaryl-Zentral-Baustein aufweisen.

In WO 2005/042458 A2 wird ein Verfahren zur kontinuierlichen Herstellung von Aldehyden beschrieben.

In WO 2008/071508 A1 wird ein Verfahren zur Hydroformylierung unter Einsatz von Bisphosphitliganden beschrieben. Unter anderem wird der Einsatz des Liganden (D-1) beschrieben.

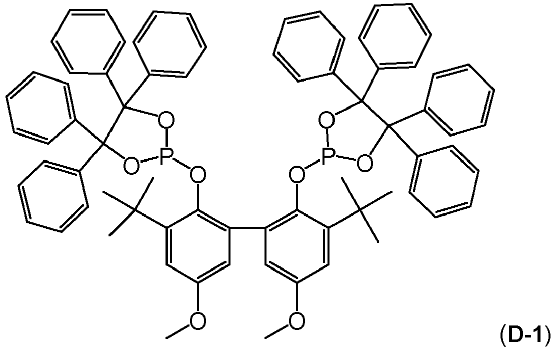

Die technische Aufgabe, welche der vorliegenden Erfindung zugrunde lag, ist die Bereitstellung neuer Verbindungen, welche gegenüber dem aus dem Stand der Technik bekannten Verbindungen bei der Hydroformylierung von Olefinen eine gesteigerte Ausbeute liefern.

Diese Aufgabe wird gelöst durch eine Verbindung nach Anspruch 1.

Verbindung gemäß Formel (**I**): 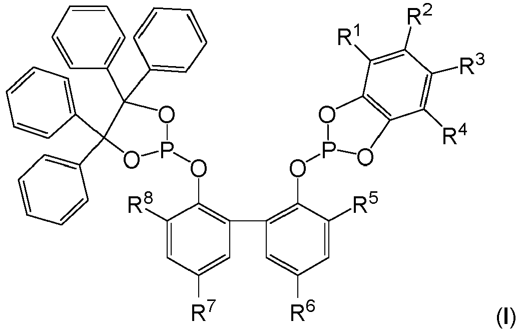 wobei
R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl.

Der Begriff -(C₁-C₁₂)-Alkyl bzw. -O-(C₁-C₁₂)-Alkyl umfasst geradkettige und verzweigte Alkylgruppen mit 1 bis 12 Kohlenstoffatomen. Vorzugsweise handelt es sich dabei um -(C₁-C₈)-Alkylgruppen bzw. -O-(C₁-C₈)-Alkylgruppen, besonders bevorzugt um -(C₁-C₄)-Alkylgruppen bzw. -O-(C₁-C₄)-Alkylgruppen.

In einer Ausführungsform stehen R⁵ und R⁸ für -(C₁-C₁₂)-Alkyl.

In einer Ausführungsform stehen R⁵ und R⁸ für -*^{ter}*Bu.

In einer Ausführungsform sind R⁶, R⁷ ausgewählt aus: -(C₁-C₁₂)-Alkyl, -O-(C₁-C₁₂)-Alkyl.

In einer Ausführungsform stehen R⁶ und R⁷ für -OCH₃ oder-*^{ter}*Bu.

In einer Ausführungsform sind R¹, R², R³, R⁴ ausgewählt aus -H, -(C₁-C₁₂)-Alkyl.

In einer Ausführungsform stehen R¹, R², R³, R⁴ für -H oder -*^{ter}*Bu.

In einer Ausführungsform weist die Verbindung eine der Strukturen (**1**) bis (**6**) auf:

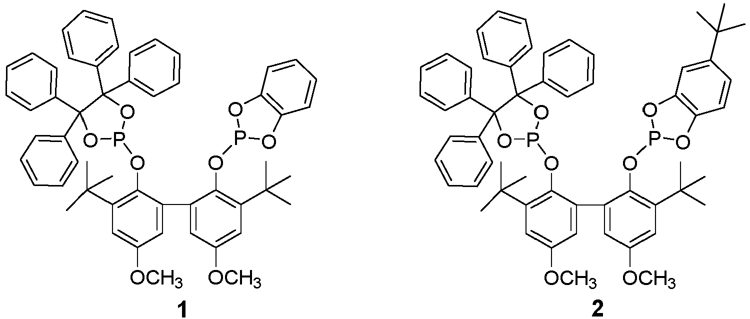

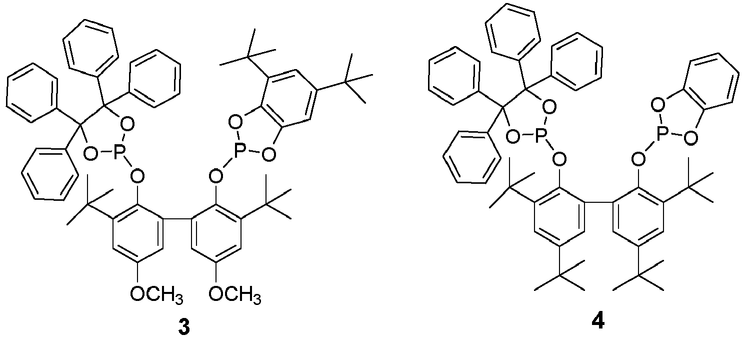

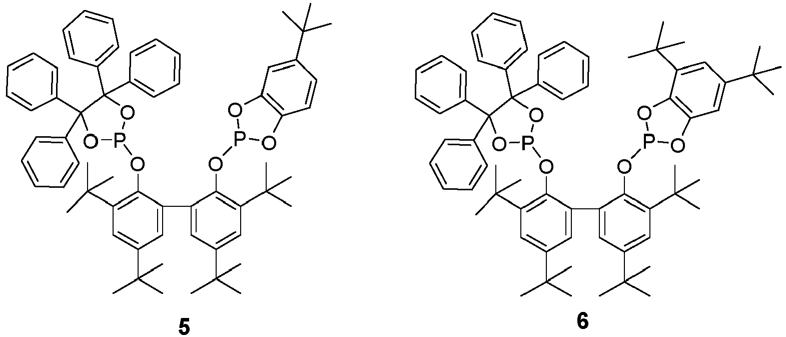

Neben der Verbindung an sich, wird auch ein Verfahren beansprucht, in welchen die Verbindung zum Einsatz kommt.

Verfahren umfassend die Verfahrensschritte:
a) Vorlegen einer ethylenisch ungesättigten Verbindung;
b) Zugabe einer wie zuvor beschriebenen Verbindung
   und einer Substanz, welche Rh umfasst;
c) Zuführen von H₂ und CO,
d) Erwärmen des Reaktionsgemisches aus a) bis c), wobei die ethylenisch ungesättigten Verbindung zu einem Aldehyd umgesetzt wird.

Hierbei können die Verfahrensschritte a), b) und c) in beliebiger Reihenfolge erfolgen. Üblicherweise erfolgt die Zugabe von CO jedoch, nachdem die Reaktionspartner in den Schritten a) und b) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

In einer Variante des Verfahrens umfasst die ethylenisch ungesättigte Verbindung keine weiteren funktionellen Gruppen außer Kohlenstoff-Kohlenstoff-Doppelbindungen.

In einer Variante des Verfahrens ist die ethylenisch ungesättigte Verbindung ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die Substanz, welche Rh umfasst, ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens erfolgt das Zuführen von CO in Verfahrensschritt c) mit einem Druck in dem Bereich von 1 bis 6 MPa (10 bis 60 bar).

In einer Variante des Verfahrens erfolgt das Erwärmen des Reaktionsgemisches in Verfahrensschritt d) auf eine Temperatur in dem Bereich von 80 °C bis 160°C.

Im Folgenden soll die Erfindung anhand von Ausführungsbeispielen näher erläutert werden.

### Synthese 2-((3,3'-Di-tert-butyl-5,5'-dimethoxy-2'-((4,4,5,5-tetraphenyl-1,3,2-dioxaphospholan-2-yl)oxy)-[1,1'-biphenyl]-2-yl)oxy)benzo[d][1,3,2]dioxaphosphol (1):

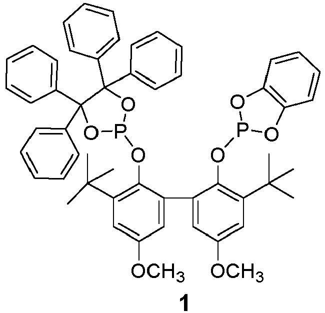

Zu einer Lösung von 2-((3,3'-Di-tert-butyl-2'-((dichlorophosphaneyl)oxy)-5,5'-dimethoxy-[1,1'-biphenyl]-2-yl)oxy)-4,4,5,5-tetraphenyl-1,3,2-dioxaphospholane (0,639 g; 0,7483 mmol) in 6 ml Toluol wird bei Raumtemperatur tropfenweise eine Mischung von Catechol (0,0824 g; 0,7483 mmol) und Triethylamin (0,42 ml) in 3 ml Toluol gegeben. Man rührt über Nacht, filtriert und engt das Filtrat im Vakuum zur Trockne ein. Der erhaltene Feststoff wird 2 h bei 60°C/ 0,1mbar getrocknet und aus 6,5 ml heißem Acetonitril aufgenommen. Der nach Abkühlen gebildete Feststoff wird abfiltriert, mit wenig kaltem Acetonitril gewaschen und im Vakuum getrocknet. Ausbeute: 0,426 g (0,5537 mmol, 74%).

Elementaranalyse (ber. für C₅₄H₅₂O₈P₂=890,945 g/Mol): C = 72,79 (72,80); H = 5,93 (5,88); P = 6,98 (6,95).

ESI-TOF HRMS: *m*/*z=* 891,3212; [M⁺+H], ber. *m*/*z=* 891,3215.

³¹P-NMR (CD₂Cl₂): d 136,0 (d, *J*_{PP}=55 Hz); 146,9 (d, *J*_{PP}=55 Hz).

¹H-NMR (CD₂Cl₂): d 1,29 (s, 9H); 1,35 (s, 9H); 3,74 (s, 3H); 3,80 (s, 3H); 6,75 (m, 1H); 6,82 (m, 1 H); 6,95-7,16 (m, 22H); 7,32 (m, 4H) ppm.

### Synthese 5-(tert-butyl)-2-((3,3'-di-tert-butyl-5,5'-dimethoxy-2'-((4,4,5,5-tetraphenyl-1,3,2-dioxaphospholan-2-yl)oxy)-[1,1'-biphenyl1-2-yl)oxy)benzo[d][1,3,2]dioxaphosphol (2):

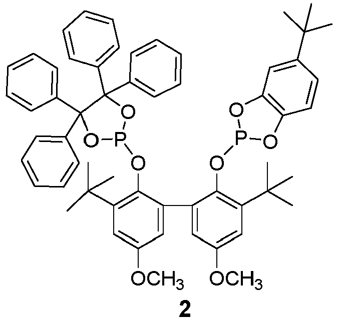

Zu einer Lösung von 2-((3,3'-Di-tert-butyl-2'-((dichlorophosphaneyl)oxy)-5,5'-dimethoxy-[1,1'-biphenyl]-2-yl)oxy)-4,4,5,5-tetraphenyl-1,3,2-dioxaphospholane (0,4384 g; 0,5135 mmol) in 4 ml Toluol wird bei Raumtemperatur tropfenweise eine Mischung von 4-tert-Butylcatechol (0,0853 g; 0,5135 mmol) und Triethylamin (0,29 ml) in 2 ml Toluol gegeben. Man rührt über Nacht, filtriert und engt das Filtrat im Vakuum zur Trockne ein. Der erhaltene Feststoff wird 2h bei 60°C/ 0,1mbar getrocknet und in 4,3 ml heißem Acetonitril aufgenommen. Der nach Abkühlen gebildete Feststoff wird abfiltriert, mit wenig kaltem Acetonitril gewaschen und im Vakuum getrocknet. Ausbeute: 0,201 g (0,261 mmol, 51%).

Elementaranalyse (ber. für C₅₈H₆₀O₈P₂=947,052 g/Mol): C = 73,51 (73,56); H = 6,63 (6,39); P = 6,81 (6,54).

ESI-TOF HRMS: *m*/*z*=696,3655; [M⁺+Na], ber. *m*/*z*=696,3660.

³¹P-NMR (CD₂Cl₂): d 135,7 (d, *J*_{PP}=44 Hz); 136,0 (d, *J*_{PP}=55 Hz).; 145,0 (d, *J*_{PP}=55 Hz); 145,1 (d, *J*_{PP}=44 Hz) ppm.

¹H-NMR (CD₂Cl₂): d 1,29+1,31 (2s, 9H); 1,33+1,34 (2s, 9H); 1,35 (s; 4,5H); 1,38 (s, 4,5 H); 3,73 (s, 1,5H); 3,74 (s, 1,5H); 3,77 (s, 1,5 H); 3,80 (s, 1,5H); 6,73 (m, 1H); 6,82 (m, 1H); 6,90-7,13 (m, 21H); 7,27-7,37 (m, 4H) ppm.

### Synthese 4,6-Di-tert-butyl-2-((3,3',Di-tert-butyl-5,5'-dimethoxy-2'-((4,4,5,5-tetraphenyl-1,3,2-dioxaphospholan-2-yl)oxy)-[1,1'-biphenyl]-2-yl)oxy)benzo[d][1,3,2]dioxaphosphol (3):

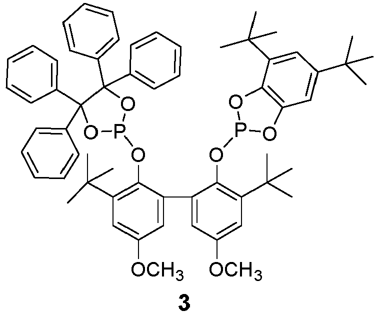

Zu einer Lösung des L1 in 4 ml THF wird bei -20°C tropfenweise die Lösung des n-BuLi gegeben. Man rührt noch weitere 20 min, lässt dann auf Raumtemperatur erwärmen und gibt tropfenweise das Benzopinakol-Phosphorchloridit, gelöst in 1,8 ml THF, zu. Die Reaktionsmischung wird über Nacht gerührt. Anschließend wird das Triethylamin-A zugesetzt und dann zu der auf 0 °C gekühlten Reaktionsmischung eine Lösung des Phosphortrichlorides in 1,5 ml THF getropft. Man lässt auf Raumtemperatur erwärmen und rührt für 6 h.

Man entfernt die flüchtigen Komponenten des Ansatzes im Vakuum und trocknet den Rückstand 2 h bei 60°C und 0,1-0,5 mbar. Der erhaltene Feststoff wird in 8 ml Toluol aufgenommen. Zur resultierenden Suspension gibt man bei Raumtemperatur tropfenweise eine Mischung, bestehend aus dem 3,5-Di-tert.-catechol, Triethylamin-B und 3 ml Toluol. Man rührt über Nacht, filtriert (G4), entfernt das Lösungsmittel im Vakuum und trocknet den Feststoff bei 60 °C und 0,1-0,5 mbar. Rohausbeute: 1,03 g (95%).

Das Rohprodukt wird in 11 ml siedendem Acetonitril gelöst. Man lässt zunächst langsam auf Raumtemperatur abkühlen und lagert dann über Nacht bei -30°C. Der abgeschiedene Feststoff wird unter Kühlung auf-30 °C durch Abhebern der überstehenden Mutterlauge mittels Tauchfritte isoliert, und anschließend im Vakuum 5 h bei 60 °C getrocknet. Ausbeute: 0,786 g (72%).

Elementaranalyse (ber. für C₆₂H₆₈O₈P₂= 1003,16 g/Mol): C = 74,24 (74,23); H = 6,85 (6,83); P = 6,07 (6,18).

ESI-TOF HRMS: *m*/*z=* 1025,4309; [M⁺+H], ber. *m*/*z=* 1025,4287.

³¹P-NMR (CD₂Cl₂): d 134,1 (s, br); 134,5 (d, *J*_{PP}= 77,3 Hz); 144,6 (d, *J*_{PP}= 77,3 Hz); 145,2 (d, *J*_{PP}= 24,7 Hz), Gemisch von zwei Diastereomeren.

¹H-NMR (CD₂Cl₂): d 1,22 (s); 1,27 (s); 1,33 (s); 1,34 (s); 1,36 (s); 1,44 (s); 1,46 (s); 1,47 (s) ppm; S=36H. 3,68 (s); 3,69 (s); 3,77 (s); 3,87 (s) ppm; S=12H. 6,59-7,43 ppm (m, 26 H).

### Synthese 2-((3,3',5,5'-tetra-tert-butyl-2'-((4,4,5,5-tetraphenyl-1,3,2-dioxaphospholan-2-yl)oxy)-[1,1'-biphenyl]-2-yl)oxy)benzo[d][1,3,2]dioxaphosphole (4):

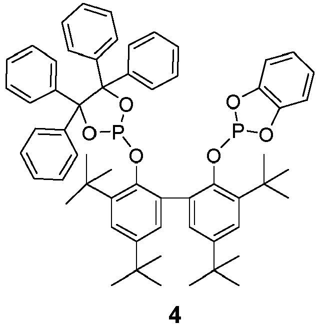

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholane (0,7936 g; 0,8760 mmol) in 5 ml Toluol wird bei Raumtemperatur tropfenweise eine Mischung von von Catechol (0,0964 g; 0,8760 mmol) und Triethylamin (0,49 ml) in 3 ml Toluol gegeben. Man rührt über Nacht, filtriert und engt das Filtrat im Vakuum zur Trockne ein. Der erhaltene Feststoff wird 2 h bei 60°C/ 0,1mbar getrocknet und anschließend 1 h in 7 ml Acetonitril verrührt. Der verbliebene Feststoff wird abfiltriert, mit wenig kaltem Acetonitril gewaschen und im Vakuum getrocknet. Ausbeute: 0,6197 g (0,657 mmol, 75%).

Elementaranalyse (ber. für C₆₀H₆₄O₆P₂=943,1076 g/Mol): C = 76,48 (76,41); H = 6,84 (6,84); P = 6,57 (6,57).

ESI-TOF HRMS: *m*/*z*=965,4076; [M⁺+Na], ber. *m*/*z*=965,4070.

³¹P-NMR (CD₂Cl₂): d 134,4 (d, *J*_{PP}=13 Hz); 145,6 (d, *J*_{PP}=13 Hz) ppm.

¹H-NMR (CD₂Cl₂): d 1,25 (s, 9H); 1,38 (s, 9H); 1,46 (s, 9H); 1,48 (s, 9H); 6,64 (m, 2 H); 6,85 (m, 1H); 6,99-7,13 (m, 19H); 7,33-7,38 (m, 3H); 7,44 (m, 1H), 7,67(m, 2H) ppm.

### Synthese 5-(tert-Butyl)-2-((3,3'-di-tert-butyl-5,5'-dimethoxy-2'-((4,4,5,5-tetraphenyl-1,3,2-dioxaphospholan-2-yl)oxy)-[1,1'-biphenyl1-2-yl)oxy)benzo[d][1,3,2]dioxaphosphol (5):

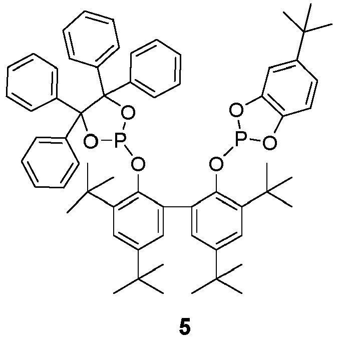

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholan (0,618 g; 0,682 mmol) in 5 ml Toluol wird bei Raumtemperatur tropfenweise eine Mischung von 4-tert-Butylcatechol (0,1133 g; 0,6818 mmol) und Triethylamin (0,38 ml) in 3 ml Toluol gegeben. Man rührt über Nacht, filtriert und engt das Filtrat im Vakuum zur Trockne ein. Der erhaltene Feststoff wird 2 h bei 60°C/ 0,1mbar getrocknet und in 6 ml heißem Acetonitril aufgenommen. Der nach Lagerung der Lösung im Tiefkühlschrank gebildete Feststoff wird abfiltriert, mit wenig kaltem Acetonitril gewaschen und im Vakuum getrocknet. Ausbeute: 0,416 g (0,477 mmol, 70%).

Elementaranalyse (ber. für C₆₄H₇₂O₆P₂=999,215 g/Mol): C = 76,75 (76,93); H = 7,20 (7,26); P = 6,15 (6,20).

ESI-TOF HRMS: *m*/*z*=1021,4708; [M⁺+Na], ber. *m*/*z*=1021,4696.

³¹P-NMR (CD₂Cl₂): d 136,2 (d, *J*_{PP}=10 Hz); 136,3 (d, *J*_{PP}=10 Hz); 145,7 (d, *J*_{PP}=10 Hz); 145,8 (d, *J*_{PP}=10 Hz) ppm. 2 Diastereomere.

¹H-NMR (CD₂Cl₂): d 1,24+1,25 (2s, 9H); 1,35+1,37+1,38 (3s, 18H); 1,45+1,48+1,49 (3s, 18H); 6,53 (m, 2 H); 6,79 (m, 1H); 6,96-7,18 (m, 18H); 7,31-7,46 (m, 4H); 7,64 (t; *J*_{HH}=2,3 Hz; 1H), 7,67(d; *J*_{HH}=2,5 Hz; 1H) ppm.

### Synthese 4,6-Di-tert-butyl-2-((3,3',5,5'-tetra-tert-butyl-2'-((4,4,5,5-tetraphenyl-1,3,2-dioxaphospholan-2-yl)oxy)-[1,1'-biphenyl1-2-yl)oxy)benzo[d][1,3,2]dioxaphosphol (6):

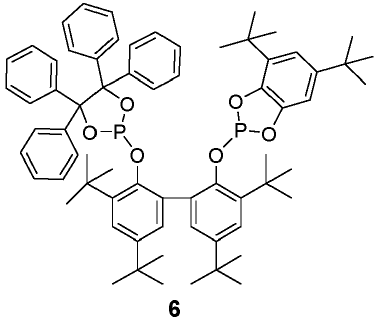

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholan (0,6529 g; 0,7207 mmol) in 5 ml Toluol wird bei Raumtemperatur tropfenweise eine Mischung von von 3,5-Ditert-Butylcatechol (0,1602 g; 0,7207 mmol) und Triethylamin (0,40 ml) in 3 ml Toluol gegeben. Man rührt über Nacht, filtriert und engt das Filtrat im Vakuum zur Trockne ein. Der erhaltene Feststoff wird 2 h bei 60°C/ 0,1mbar getrocknet, dann 1,5 h in 7 ml Acetonitril verrührt und nach Filtration im Vakuum getrocknet. Ausbeute: 0,5345 g (0,5064 mmol, 70%). Elementaranalyse (ber. für C₆₈H₈₀O₆P₂₌1055,322 g/Mol): C = 77,49 (77,39); H = 7,57 (7,64); P = 5,90 (5,87).

ESI-TOF HRMS: *m*/*z*=1077,5305; [M⁺+Na], ber. *m*/*z*=1077,5322.

³¹P-NMR (CD₂Cl₂): d 132,5 (d, *J*_{PP}=20 Hz); 134,4 (s, br); 144,3 (d, *J*_{PP}=20 Hz); 145,3 (d, *J*_{PP}=11 Hz) ppm. 2 Diastereomere.

¹H-NMR (CD₂Cl₂): d 1,19 (s; 4,5H); 1,24 (s; 4,5H); 1,31 (s; 4,5H); 1,33 (s; 4,5H); 1,34 (s; 4,5H); 1,37 (s; 4,5H); 1,41 (s; 4,5H); 1,44 (s; 4,5H); 1,45 (s, 9H); 1,47 (s; 4,5H); 1,49 (s, 4,5H); 6,42 (m, 1H); 6,73 (m; 0,5H); 6,85 (dd, *J*_{HH}=20,3 Hz; *J*_{HH}=1,99 Hz; 1H); 6,96-7,16 (m, 16H); 7,23-7,30 (m; 1,5H); 7,31-7,43 (m, 4H); 7,60 (dd, *J*_{HH}=12,5 Hz; *J*_{HH}=2,4 Hz; 1H), 7,66 (dd, *J*_{HH}=8,5 Hz; *J*_{HH}=2,5 Hz; 1H) ppm.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac=Acetylacetonat-Anion; COD = 1,5-Cyclooctadien), zum Einsatz. Der Autoklav wurde bei 12 bar für den Enddruck von 20 bar unter Rühren (1500 U/min) aufgeheizt. Nach Erreichen der Reaktionstemperatur wurde das Olefin mit einem in der Druckpipette eingestellten Überdruck in den Autoklaven gepresst. Die Reaktion wurde bei konstantem Druck (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

Die Reaktion wurde mit den erfindungsgemäßen Verbindungen (1) bis (6), sowie mit dem Vergleichsliganden (D-1) durchgeführt.

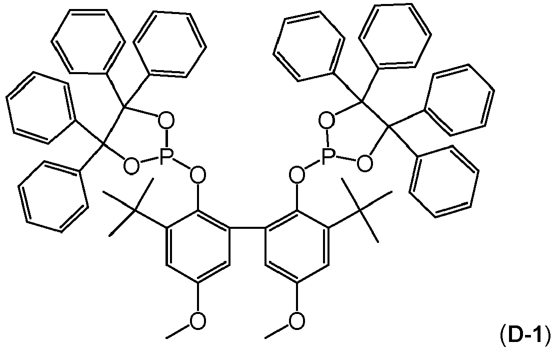

### Reaktionsbedingungen:

Olefin: 2-Penten, Solvens: Toluol, Masseanteil Rhodium: 100 ppm, p: 20 bar, T: 120 °C, t: 4 h, Verhältnis Rh : Ligand = 1 : 2.

Die Ergebnisse sind in der nachfolgenden Tabelle zusammengestellt:

| Ligand | Ausbeute Aldehyd [%] |
|---|---|
| **1*** | 99 |
| **2*** | 99 |
| **3*** | 99 |
| **4*** | 99 |
| **5*** | 99 |
| **6*** | 99 |
| **D-1** | 14 |

| | |
|---|---|
| *erfindungsgemäße Verbindung | |

Wie die Versuchsergebnisse zeigen, wird die Aufgabe durch die erfindungsgemäßen Verbindungen gelöst.

## Patentansprüche

1. Verbindung gemäß Formel (**I**): 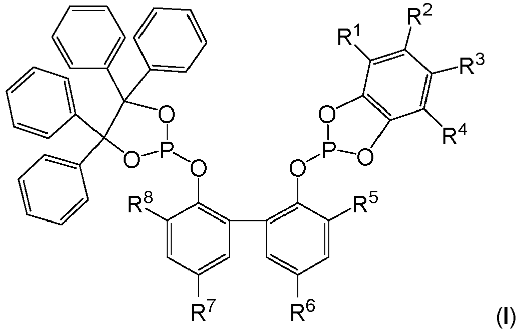 wobei
R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl.

2. Verbindung nach Anspruch 1,
wobei R⁵ und R⁸ für -(C₁-C₁₂)-Alkyl stehen.

3. Verbindung nach einem der Ansprüche 1 oder 2,
wobei R⁵ und R⁸ für -*^{ter}*Bu stehen.

4. Verbindung nach einem der Ansprüche 1 bis 3,
wobei R⁶, R⁷ ausgewählt sind aus: -(C₁-C₁₂)-Alkyl, -O-(C₁-C₁₂)-Alkyl.

5. Verbindung nach einem der Ansprüche 1 bis 4,
wobei R⁶ und R⁷ für -OCH₃ oder-*^{ter}*Bu stehen.

6. Verbindung nach einem der Ansprüche 1 bis 5,
wobei R¹, R², R³, R⁴ ausgewählt sind aus -H, -(C₁-C₁₂)-Alkyl.

7. Verbindung nach einem der Ansprüche 1 bis 8,
wobei R¹, R², R³, R⁴ für -H oder -*^{ter}*Bu stehen.

8. Verbindung nach einem der Ansprüche 1 bis 7,
wobei die Verbindung eine der Strukturen (**1**) bis (**6**) aufweist:

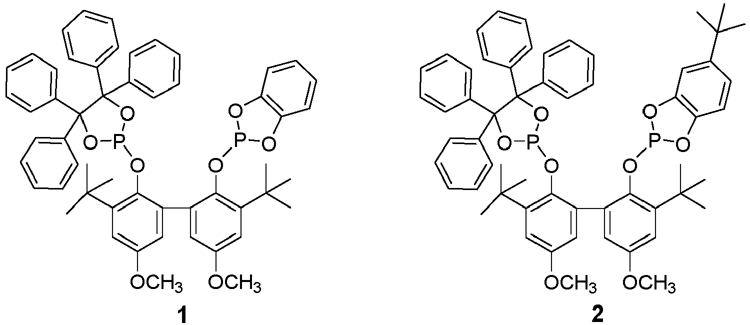

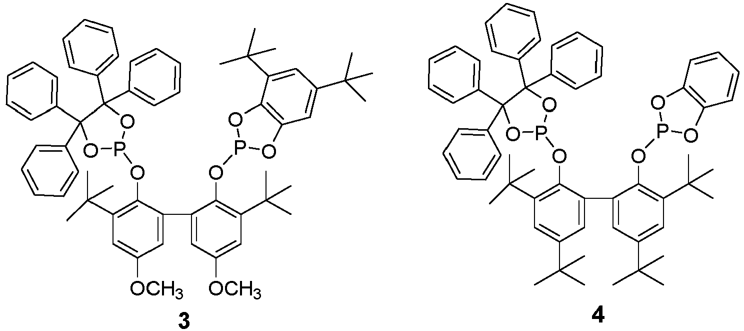

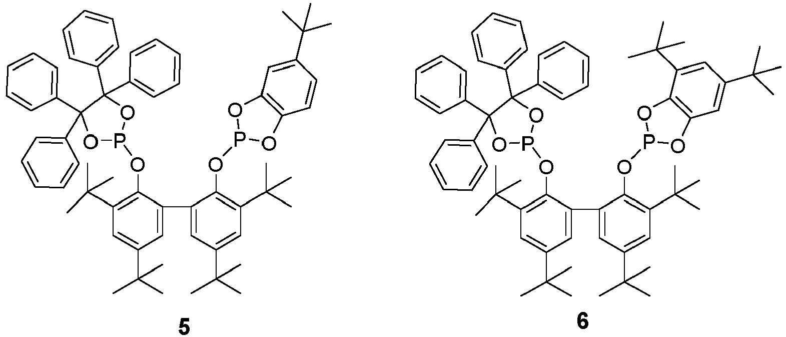

9. Verfahren umfassend die Verfahrensschritte:
a) Vorlegen einer ethylenisch ungesättigten Verbindung;
b) Zugabe einer Verbindung gemäß einem der Ansprüche 1 bis 8 und einer Substanz, welche Rh umfasst;
c) Zuführen von H₂ und CO,
d) Erwärmen des Reaktionsgemisches aus a) bis c), wobei das Olefin zu einem Aldehyd umgesetzt wird.

10. Verfahren nach Anspruch 9,
wobei die ethylenisch ungesättigte Verbindung in Verfahrensschritt a) ausgewählt ist aus: Ethen, Propen, 1-Buten, *cis-* und/oder t*rans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

11. Verfahren nach einem der Ansprüche 9 oder 10,
wobei die Substanz, welche Rh umfasst, ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

12. Verfahren nach einem der Ansprüche 9 bis 11,
wobei das Zuführen von CO in Verfahrensschritt c) mit einem Druck in dem Bereich von 1 bis 6 MPa (10 bis 60 bar) erfolgt.

13. Verfahren nach einem der Ansprüche 9 bis 12,
wobei das Erwärmen des Reaktionsgemisches in Verfahrensschritt d) auf eine Temperatur in dem Bereich von 80 °C bis 160 °C erfolgt.

## Claims

1. Compound of formula (I): 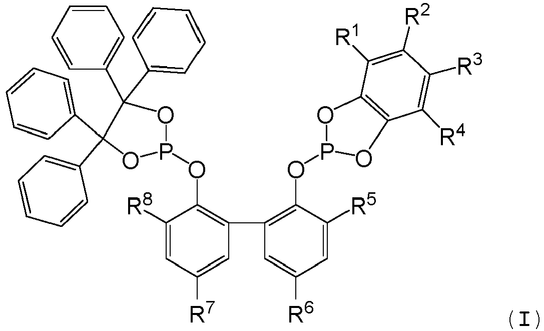 wherein
R¹, R², R³, R⁴, R⁵, R⁶, R⁷, R⁸ are each independently selected from: -H, -(C₁-C₁₂)-alkyl, -O-(C₁-C₁₂) -alkyl.

2. Compound according to Claim 1,
wherein R⁵ and R⁸ are -(C₁-C₁₂) -alkyl .

3. Compound according to either of Claims 1 and 2,
wherein R⁵ and R⁸ are -*^{tert}*Bu.

4. Compound according to any of Claims 1 to 3,
wherein R⁶, R⁷ are selected from: -(C₁-C₁₂) -alkyl, -O-(C₁-C₁₂)-alkyl.

5. Compound according to any of Claims 1 to 4,
wherein R⁶ and R⁷ are -OCH₃ or -*^{tert}*Bu.

6. Compound according to any of Claims 1 to 5,
wherein R¹, R², R³, R⁴ are selected from -H, -(C₁-C₁₂)-alkyl.

7. Compound according to any of Claims 1 to 8,
wherein R¹, R², R³, R⁴ are -H or -*^{tert}*Bu.

8. Compound according to any of Claims 1 to 7,
wherein the compound has one of the structures (1) to (**6**):

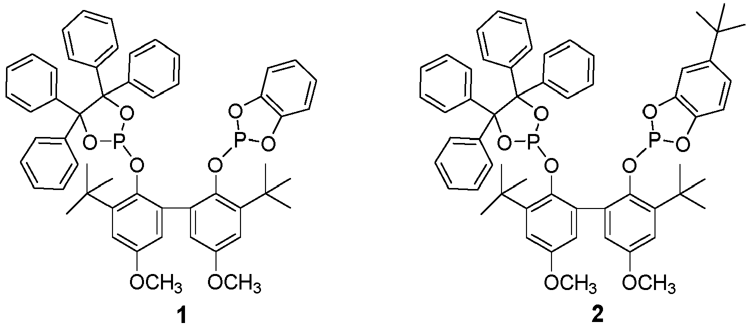

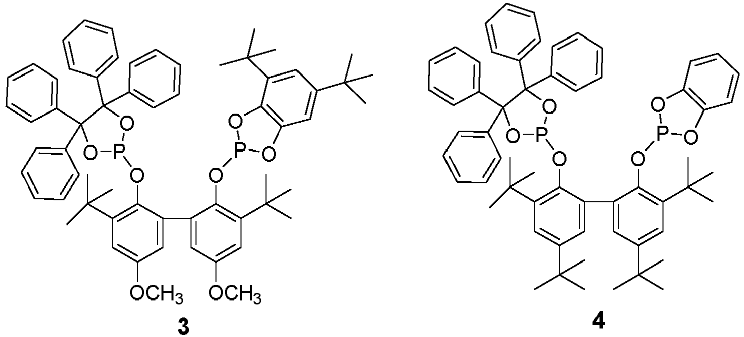

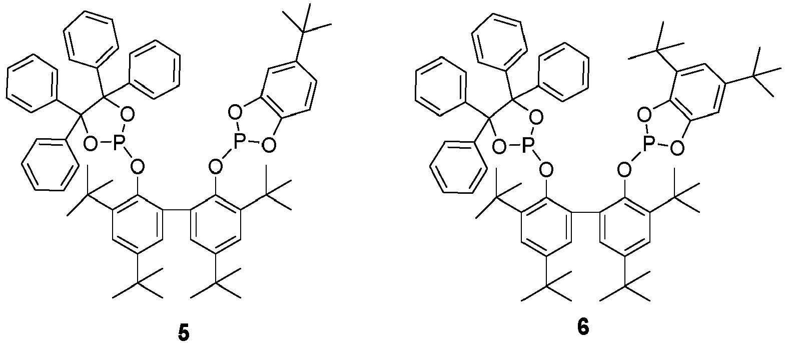

9. Process comprising the process steps of:
a) initially charging an ethylenically unsaturated compound;
b) adding a compound according to any of Claims 1 to 8
and a substance comprising Rh;
c) feeding in H₂ and CO,
d) heating the reaction mixture from a) to c), with conversion of the olefin to an aldehyde.

10. Process according to Claim 9,
wherein the ethylenically unsaturated compound in process step a) is selected from: ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, or mixtures thereof.

11. Process according to either of Claims 9 and 10,
wherein the substance comprising Rh is selected from: Rh(acac)(CO)₂, [(acac)Rh(COD)] (Umicore, acac = acetylacetonate anion; COD = 1,5-cyclooctadiene), Rh₄CO₁₂.

12. Process according to any of Claims 9 to 11,
wherein CO is fed in in process step c) at a pressure in the range from 1 to 6 MPa (10 to 60 bar).

13. Process according to any of Claims 9 to 12,
wherein the reaction mixture is heated in process step d) to a temperature in the range from 80°C to 160°C.

## Revendications

1. Composé selon la formule (1) : 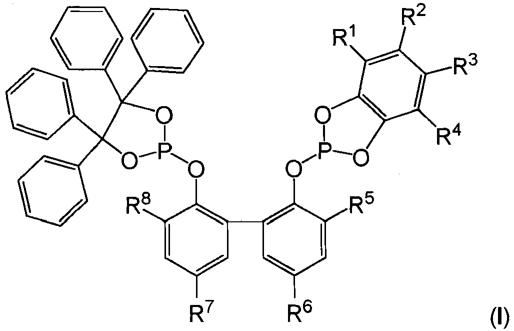 dans laquelle
R¹, R², R³, R⁴, R⁵, R⁶, R⁷ et R⁸ sont choisis, à chaque fois indépendamment les uns des autres, parmi : -H, -(C₁-C₁₂)-alkyle, -O-(C₁-C₁₂) -alkyle.

2. Composé selon la revendication 1,
R⁵ et R⁸ représentant -(C₁-C₁₂) -alkyle.

3. Composé selon l'une quelconque des revendications 1 ou 2,
R⁵ et R⁸ représentant -^{ter}Bu.

4. Composé selon l'une quelconque des revendications 1 à 3,
R⁶, R⁷ étant choisis parmi : -(C₁-C₁₂) -alkyle, -O-(C₁-C₁₂)-alkyle.

5. Composé selon l'une quelconque des revendications 1 à 4,
R⁶ et R⁷ représentant -OCH₃ ou -^{ter}Bu.

6. Composé selon l'une quelconque des revendications 1 à 5,
R¹, R², R³, R⁴ étant choisis parmi -H, -(C₁-C₁₂) -alkyle.

7. Composé selon l'une quelconque des revendications 1 à 8,
R¹, R², R³, R⁴ représentant -H ou -^{ter}Bu.

8. Composé selon l'une quelconque des revendications 1 à 7,
le composé présentant l'une des structures (1) à (6) :

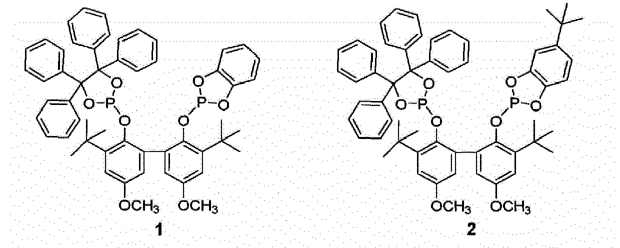

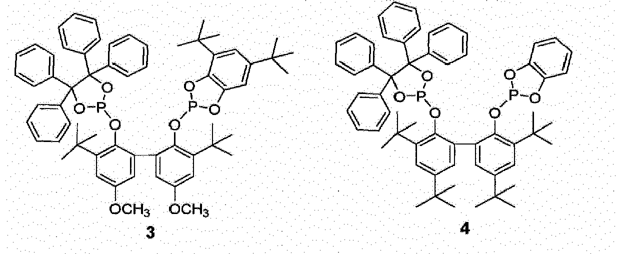

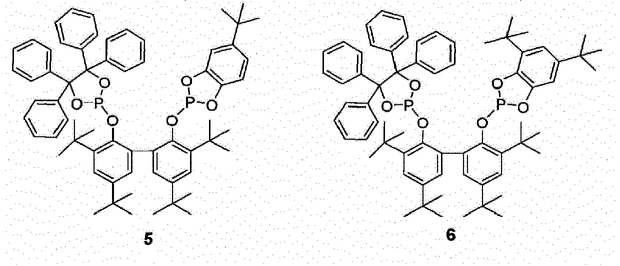

9. Procédé comprenant les étapes de procédé :
a) disposition préalable d'un composé éthyléniquement insaturé ;
b) ajout d'un composé selon l'une quelconque des revendications 1 à 8 et d'une substance qui comprend Rh ;
c) introduction de H₂ et de CO ;
d) chauffage du mélange réactionnel de a) à c), l'oléfine étant transformée en aldéhyde.

10. Procédé selon la revendication 9,
le composé éthyléniquement insaturé dans l'étape de procédé a) étant choisi parmi : l'éthylène, le propène, le 1-butène, le cis-2-butène et/ou le trans-2-butène, l'iso-butène, le 1,3-butadiène, le 1-pentène, le cis-2-pentène et/ou le trans-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

11. Procédé selon l'une quelconque des revendications 9 ou 10,
la substance qui comprend Rh étant choisie parmi : Rh(acac) (CO)₂, [(acac)Rh(COD)] (Umicore, acac = anion acétylacétonate ; COD = 1,5-cyclooctadiène), Rh₄CO₁₂.

12. Procédé selon l'une quelconque des revendications 9 à 11,
l'introduction de CO dans l'étape de procédé c) ayant lieu à une pression dans la plage de 1 à 6 MPa (10 à 60 bars).

13. Procédé selon l'une quelconque des revendications 9 à 12,
le chauffage du mélange réactionnel dans l'étape de procédé d) ayant lieu à une température dans la plage de 80 à 160°C.
